(19) **Europäisches Patentamt / European Patent Office / Office européen des brevets**

(11) **EP 3 795 078 A1**

(12) **EUROPEAN PATENT APPLICATION**

(43) Date of publication:
24.03.2021 Bulletin 2021/12

(21) Application number: 20197183.5

(22) Date of filing: 21.09.2020

(51) Int Cl.:
*A61B 5/318* (2021.01)    *A61B 5/0538* (2021.01)
*A61B 5/00* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 22.09.2019  US 201962903850 P
10.09.2020  US 202017016608

(71) Applicant: Biosense Webster (Israel) Ltd
Yokneam, 2066717 (IL)

(72) Inventors:
• AMIT, Matityahu
2066717 Yokneam (IL)
• TSOREF, Liat
2066717 Yokneam (IL)
• AMOS, Yariv Avraham
2066717 Yokneam (IL)
• SHALGI, Avi
2066717 Yokneam (IL)

(74) Representative: Small, Gary James
Carpmaels & Ransford LLP
One Southampton Row
London WC1B 5HA (GB)

(54) **GUIDING CARDIAC ABLATION USING MACHINE LEARNING (ML)**

(57) A system includes an interface and a processor. The interface is configured to receive data that characterizes an initial ablation operation applied to a region of a heart of a patient. The processor is configured to automatically specify, based on the received data, if found required, a complementary ablation operation to be applied to the region.

EP 3 795 078 A1

**Description**

**CROSS-REFERENCE TO RELATED APPLICATIONS**

This application claims the benefit of U.S. Provisional Patent Application 62/903,850, filed September 22, 2019, whose disclosure is incorporated herein by reference.

**FIELD OF THE INVENTION**

The present invention relates generally to processing of electrophysiological signals and ablation, and specifically to optimizing cardiac ablation parameters using machine learning (ML).

**BACKGROUND OF THE INVENTION**

Methods and systems for planning and guiding, based on patient data, an ablation procedure to treat cardiac arrhythmia were previously reported in the patent literature. For example, United States Patent No. 9,463,072 describes a method and system for patient-specific planning and guidance of electrophysiological interventions. A patient-specific anatomical heart model is generated from cardiac image data of a patient. A patient-specific cardiac electrophysiology model is generated based on the patient-specific anatomical heart model and patient-specific electrophysiology measurements. Virtual electrophysiological interventions are performed using the patient-specific cardiac electrophysiology model. A simulated electrocardiogram (ECG) signal is calculated in response to each virtual electrophysiological intervention. Embodiments of the invention utilize advanced machine learning algorithms, a LBM-EP (Lattice-Boltzmann Method for Electrophysiology) technique for near real time modeling of cardiac electrophysiology, and a model of generation of ECG signals to predict and display patient-specific electrocardiograms after virtual EP therapies.

As another example, U.S. Patent No. 9,277,970 describes a method and system for patient-specific planning and guidance of an ablation procedure for cardiac arrhythmia. A patient-specific anatomical heart model is generated based on pre-operative cardiac image data. The patient-specific anatomical heart model is registered to a coordinate system of intra-operative images acquired during the ablation procedure. One or more ablation site guidance maps are generated based on the registered patient-specific anatomical heart model and intra-operative patient-specific measurements acquired during the ablation procedure. The ablation site guidance maps may include myocardium diffusion and action potential duration maps. The ablation site guidance maps are generated using a computational model of cardiac electrophysiology which is personalized by fitting parameters of the cardiac electrophysiology model using the intra-operative patient-specific measurements. The ablation site guidance maps are displayed by a display device during the ablation procedure. In an embodiment, registering the patient-specific anatomical heart model to an intra-operative three-dimensional rotational angiography image acquired during the ablation procedure comprises calculating a probability map of a cardiac pericardium in the three-dimensional rotational angiography image using a machine learning algorithm.

**SUMMARY OF THE INVENTION**

An embodiment of the present invention that is described hereinafter provides a system including an interface and a processor. The interface is configured to receive data that characterizes an initial ablation operation applied to a region of a heart of a patient. The processor is configured to automatically specify, based on the received data, if found required, a complementary ablation operation to be applied to the region.

In some embodiments, the processor is configured to specify the complementary ablation by assessing a quality of the initial ablation operation, and specifying the complementary ablation operation in response to finding that the quality of the initial ablation operation does not meet a quality criterion.

In some embodiments, the data that characterizes the initial ablation operation includes at least one of a lesion depth; a lesion radius; a lesion major axis; a lesion minor axis; a lesion 3D location; a lesion anatomical location; and a lesion surface area.

In an embodiment, in specifying the complementary ablation operation, the processor is configured to specify a location for a repeat ablation.

In another embodiment, in specifying the complementary ablation operation, the processor is configured to indicate a gap in a segment of ablation points.

In yet another embodiment, in specifying the complementary ablation operation, the processor is configured to specify, in real time, that an additional ablation is to be performed in proximity to a segment of ablation points.

In some embodiments, in specifying the complementary ablation operation, the processor is further configured to specify values of one or more ablation parameters to be used in the complementary ablation.

In some embodiments, the data that characterizes the initial ablation operation includes at least one of a body surface electrocardiogram (ECG) signal; a change in a body surface ECG signal; an intra-cardiac ECG signal; a change in an intra-cardiac ECG signal; an impedance of an ablation electrode; a change in an impedance of an ablation electrode; a temperature of ablated tissue; a change of temperature of ablated tissue; a force on ablated tissue; a change of force on ablated tissue; an ablation catheter type; a 3D location of an ablation point; a predicted anatomical location of an ablation point; an ablation duration of an ablation point; a rate of irrigation; and a power delivered during an ablation.

In other embodiments, the data that characterizes the initial ablation operation comprises one or both of a change in ultrasound reflection of ablated tissue, and a change in a magnetic resonance image (MRI) of ablated tissue.

In some embodiments, the processor is configured to automatically specify the complementary ablation operation by applying a trained machine learning (ML) model.

In an embodiment, the ML model includes at least one of an autoencoder, a variational autoencoder, a general adversarial network (GAN), a random forest (RF), a supervised ML, and a reinforcement ML.

There is additionally provided, in accordance with another embodiment of the present invention, a method including receiving data that characterizes an initial ablation operation applied to a region of a heart of a patient. If found required, a complementary ablation operation to be applied to the region is automatically specified, by a processor, based on the received data.

## BRIEF DESCRIPTION OF THE DRAWINGS

The present invention will be more fully understood from the following detailed description of the embodiments thereof, taken together with the drawings in which:

Fig. 1 is a schematic, pictorial illustration of a catheter-based electrophysiological (EP) sensing, signal-analysis, and IRE ablation system, according to an exemplary embodiment of the present invention;

Fig. 2 illustrates a deep learning algorithm for lesion estimation based on autoencoder and a random forest (RF), according to an exemplary embodiment of the present invention;

Fig. 3 is a flow chart of training and use for inference, of a machine learning (ML) model to estimate and correct ablation results, according alternative exemplary embodiments of the present invention; and

Fig. 4 is screen shot of visualizations of pulmonary veins isolation (PVI) planned using the ML model of Fig. 3, according to an exemplary embodiment of the present invention.

## DETAILED DESCRIPTION OF EMBODIMENTS

OVERVIEW

Cardiac ablation is a common procedure that is used to treat arrhythmias by forming lesions in cardiac tissue of a patient. Such lesions may be formed by irreversible electroporation (IRE), or using other types of ablative energy, such as radiofrequency (RF), both of which can be applied using a catheter. In IRE ablation, the catheter is maneuvered such that electrodes disposed on a distal end of the catheter are in contact with the tissue. Then, high voltage bipolar pulses are applied between the electrodes, and strong electric field pulses produced in tissue cause cell death and lesion production. In RF ablation, an alternating RF current is applied to tissue by one or more electrodes, causing cell death by heat.

Typically, in an ablation procedure in a heart chamber to correct an arrhythmia, it is important to achieve both contiguity and transmurality (sufficient lesion depth) in the ablation. Absence of either typically leads to "leaks" of a wave front through the ablated tissue. In order to check for leaks, for example in a procedure to achieve pulmonary vein isolation (PVI), a physician paces (i.e. injects a signal into) the heart at one side of an ablation line so as to stimulate the heart, and checks to see if the signal appears at the other side. If the signal does not appear, electrical isolation, as intended, has been achieved. However, if the signal does appear then the physician typically adds ablation points.

Parameters, such as an ablation line contiguity index (ACLI), may be defined for scoring the contiguity and transmurality of an ablation line. However, such parameters can only be estimated after an ablation procedure has been completed, so that PVI can be checked only after a complete loop has been performed.

Thus, a typical current workflow may have the follow steps:

1. First "ablation loop" of PV isolation
2. Testing via stimulation
3. Ablate tissue to close gap based on stimulation; and/or
2. Testing via medicine, e.g. Adenosine, that initiates the arrhythmia
3. Ablate tissue to close the gap

It will be understood that Adenosine-challenge step 2 above may not be implemented in a daily clinic workflow, since it is generally used in a research environment.

Even if isolation is achieved during the procedure, the arrhythmia may return at some later date. In this case a "re-do" of the procedure may be necessary.

Embodiments of the present invention that are described hereinafter provide systems and machine learning (ML) methods to predict the success of a cardiac ablation procedure based only on information acquired during treatment (e.g., acquired using the ablating catheter itself), as described below. The predictions are achieved by estimating, (i) in some embodiments, lesion properties, such as to what extent the lesion is transmural, and (ii) in other embodiments, a level of contiguity and transmurality in the ablation. The later embodiments may guide a physician, in real time, in case additional ablation points are needed.

In some embodiments, a processor receives data that characterizes an initial ablation operation applied to a region of a heart of a patient. The processor automatically specifies, based on the received data, if found required, a complementary ablation operation to be applied to the region. For example, the processor assesses a quality of the initial ablation operation, and, in response to finding that the quality of the initial ablation operation does not meet a quality criterion (e.g., contiguity and/or transmurality achieved) specifies the complementary ablation operation.

Embodiments of the invention may be used to provide a recommendation for focal source and repetitive activation patterns (RAPs). Embodiments of the invention may also be used to provide an estimation of the quality of an ablation and an "optimal" ablation strategy of persistent AF drivers and perpetuators RAPs, foci, and fibrotic tissue.

In an embodiment of the invention, a machine learning (ML) model, such as an artificial neural network (ANN), is generated. The ANN model is trained using initial ablation data comprising surface electrocardiogram (ECG) signals, intra-cardiac ECG (IcECG) signals (also called electrograms (EGM), 3D location information of the data collected, as well as ablation parameters that include power used for the ablation, period of time of the ablation, temperature measured during the ablation, and impedance of the catheter electrode performing the ablation. Other parameters used to train the model include, but are not limited to, the catheter used, the force measured by the catheter, and changes in parameters such as temperature and impedance. Once the model has been generated, values of at least some of these parameters are used by the model to arrive at results formulated by the model.

In some embodiments, a ground truth, derived from clinical and preclinical data of the ablation training data, is used for training. Such data may include actual lesion parameters obtained under a range of ablative powers, including parameters such as surface area and the depth of tissue necrosis.

While performing an ablation in a new patient, a processor estimates, using an ML model, the ablated lesion, e.g., its radius and depth, which are provided to the physician. The values are typically provided on a graphic user interface (GUI), which may also provide visualization of the ablation.

In other embodiments of the invention the trained ML model identifies, after a first ablation loop, places that are potential candidates for an ablation "re-do."

Furthermore, just before and/or during a new ablation, a processor may use the model to, using the ablation data (i.e., any data acquired during ablation procedure) for the specific patient, predict levels of contiguity and transmurality in the ablation.

Using the above ML models enables a physician to reduce the time spent on a procedure, compared to that for the current workflow described above, by predicting outcomes of ablation procedures. The models enable the physician to create effective isolation by an ablation line, i.e., a line having a high contiguity and transmurality score in a first round of an ablation procedure. Thus, using the models allows a simpler, quicker and more effective process than prior art systems.

While the ANN model is used here as an example, a person skilled in the art may choose from among other ML models available for use, such as decision tree learning, support vector machines (SVM), and Bayesian networks. ANN models include, for example, convolutional NN (CNN), autoencoder, and probabilistic neural network (PNN). Typically, the one or more processors used (collectively named hereinafter "processor") are programmed in software containing a particular algorithm that enables the processor to conduct each of the processor-related steps and functions outlined above. Typically, the training is done using a computing system comprising multiple processors, such as graphics processing units (GPU) or tensor processing units (TPU). However, any of these processors may be also be central processing units (CPUs).

The ability to assess, in real time, lesion parameters (e.g., diameter, depth), as well as contiguity and transmurality in the ablation, based on limited data described above for ML algorithm inference, allows a simple assessment of the quality of the ablative treatment, may lead to a more accurate ablation profile, and typically to an improvement in outcome of the ablation procedure.

SYSTEM DESCRIPTION

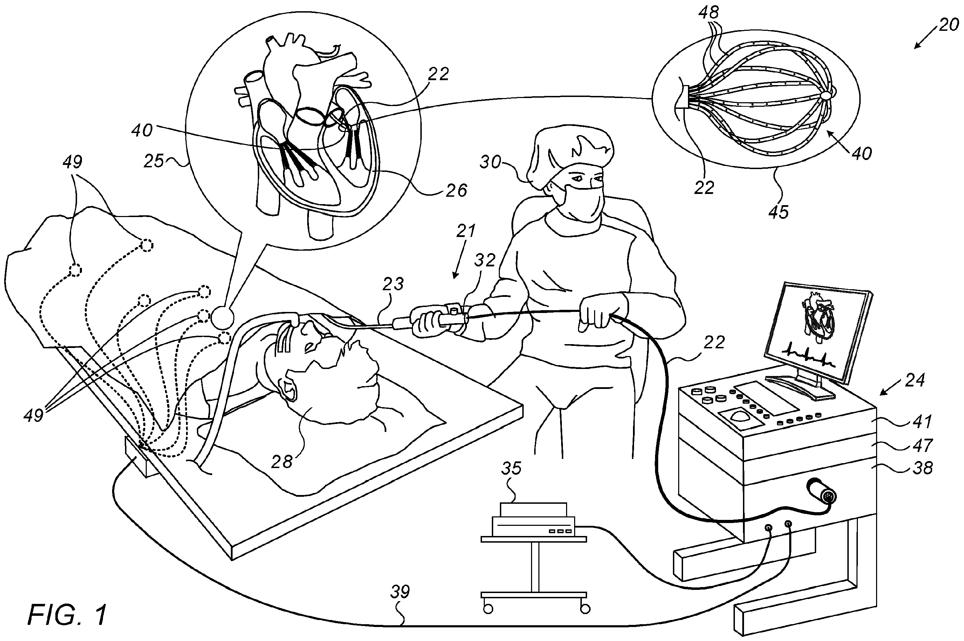

Fig. 1 is a schematic, pictorial illustration of a catheter-based electrophysiological (EP) sensing, signal-analysis, and IRE ablation system 20, according to an embodiment of the present invention. System 20 may be, for example, a CARTO® 3 system, produced by Biosense-Webster, Irvine, California. As seen, system 20 comprises a catheter 21, having a shaft 22 that is navigated by a physician 30 into a heart 26 (inset 25) of a patient 28. In the pictured example, physician 30 inserts shaft 22 through a sheath 23, while manipulating shaft 22 using a manipulator 32 near the proximal end of the catheter.

In the embodiment described herein, catheter 21 may be used for any suitable diagnostic purpose and/or tissue ablation, such as electrophysiological mapping of heart 26 and IRE ablation, respectively. An ECG recording instrument 35 may receive various types of ECG signals sensed by system 20 during the process.

As shown in inset 25, a distal end of shaft 22 of catheter 21 is fitted with a multi-electrode basket catheter 40. Inset 45 shows an arrangement of multiple electrodes 48 of basket catheter 40. The proximal end of catheter 21 is connected to a control console 24, to transmit, for example, electrograms acquired by electrodes 48.

Console 24 comprises a processor 41, typically a general-purpose computer, with suitable front end and interface circuits 38 for receiving EP signals (e.g., ECG signals) as well as non-EP signals (such as position signals) from electrodes 48 of catheter 21. For this purpose, processor 41 is connected to electrodes 48 via wires running within shaft 22. Interface circuits 38 are further configured to receive ECG signals, such as from a multichannel (e.g., 12-lead) ECG apparatus that can be ECG recording instrument 35, as well as non-ECG signals from surface body electrodes 49. Typically, electrodes 49 are attached to the skin around the chest and legs of patient 28. Processor 41 is connected to electrodes 49 by wires running through a cable 39 to receive signals from electrodes 49.

Four of surface body electrodes 49 are named according to standard ECG protocols: MA (right arm), LA (left arm), ML (right leg), and LL (left leg). A Wilson Central Terminal (WCT) may be formed by three of the four named body surface electrodes 49, and a resulting ECG signal, $V_{WCT}$, is received by interface circuits 38.

During an EP mapping procedure, the locations of electrodes 48 are tracked while they are inside heart 26 of the patient. For that purpose, electrical signals are passed between electrodes 48 and body surface electrodes 49. Based on the signals, and given the known positions of electrodes 22 on the patient's body, a processor 41 calculates an estimated location of each electrode 22 within the patient's heart. Such tracking may be performed using the Active Current Location (ACL) system, made by Biosense-Webster (Irvine California), which is described in US Patent No. 8,456,182, whose disclosure is incorporated herein by reference.

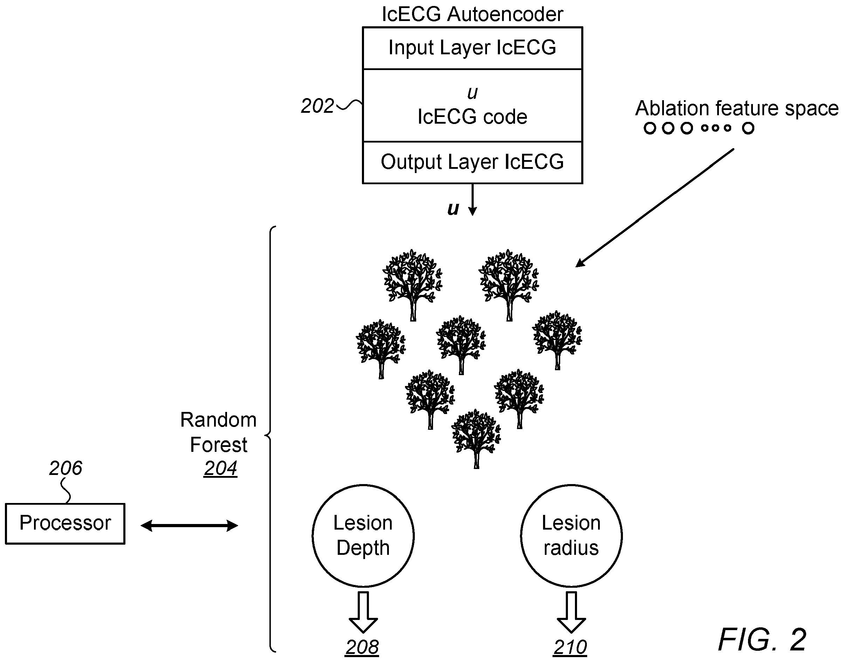

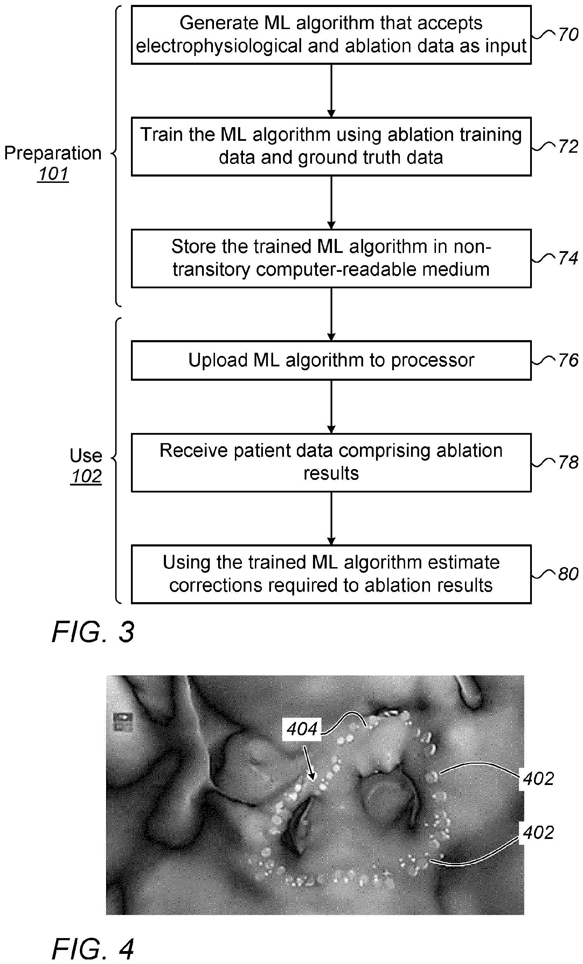

The processor may thus associate any given signal received from electrodes 48, such as EGMs, with the location at which the signal was acquired. Processor 41 uses information contained in these signals to construct an EP map, such as a local activation time (LAT) map, to present on a display. In the shown embodiment, using an algorithm comprising an ML algorithm applied to EP and other data (e.g., irrigation rate), as described in Figs. 2 and 3, processor 41 estimates contiguity and transmurality of lesions the system ablates.

To perform IRE ablation, electrodes 48 are connected (e.g., switched) to an IRE pulse generator 47 comprising a processor-controlled switching circuitry (e.g., an array of relays, not shown) in console 24. Using estimations provided by the disclosed technique, such as a level of ablation contiguity, processor 41, or the physician, may select which electrodes to connect to pulse-generator 37 to apply IRE pulses (via the switching circuitry).

During IRE ablation, initial ablation data defined below may be used in inference by one of the above ML models, to further assess (e.g., in real time) lesion parameters, as described in Fig. 2, and contiguity and transmurality of a group of lesions.

Processor 41 is typically programmed in software to carry out the functions described herein. The software may be downloaded to the processor in electronic form, over a network, for example, or it may, alternatively or additionally, be provided and/or stored on non-transitory tangible media, such as magnetic, optical, or electronic memory. In particular, processor 41 runs a dedicated algorithm as disclosed herein, such as included in Fig. 3, that enables processor 41 to perform the disclosed steps, as further described below.

GUIDING CARDIAC ABLATION USING ML

Fig. 2 is an illustration of operation of a machine-learning (ML) model, according to an exemplary embodiment of the present invention. The model is built from random forest regression trees based on ablation training data described below. A random forest classifier 204 is a committee of decision trees, where each decision tree has been fed a subset of the attributes of data and predicts on the basis of that subset. The mode of the actual predicted values of the decision trees are taken into account to provide ultimate random forest answers 208 and 210. The random forest classifier, generally, alleviates overfitting, which is present in a standalone decision tree, leading to a much more robust and accurate classifier.

The model is based on deep learning autoencoders as well as on the random forest regression trees. The autoencoders perform a dimensionality reduction to a set of features u that later serve as a feature space for lesion assessment.

Autoencoder 202 comprises two parts: an encoder and a decoder. The encoder maps an input (herein an ECG signal and/or an IcECG signal) to a hidden representation (u) via a nonlinear transformation. Then, the decoder maps the hidden representation back to reconstructed data via another nonlinear transformation:

$$u = f(IcECG, \theta_{encoder}), \qquad IcECG = g(u, \varphi_{decoder}).$$

Embodiments of the invention use the same network architecture for ECG and IcECG reconstruction. An L2 normalization function is minimized in order to learn a set of $\theta_{encoder}$, $\varphi_{decoder}$ weights to reconstruct IcECG (or body surface ECG). The autoencoder is implemented using a fully connected convolutional neural network (FCN) of an encoder and a decoder with a predefined number of layers. A random forest regression is then performed based on the encoded representation u, medical history (e.g. AF duration, NYHA score) parameters of the patient, demographic of the patient (e.g., age, BMI) and ablation features (e.g. power, temperature profile) in order to predict lesion depth.

In the embodiment, the model uses an ablation feature space as an input layer Ablation feature space as an input to the random forest. The ablation feature space refers to ablation characteristics (e.g., power, impedance, impedance drop, stability, ablation Index and x,y,z position of each ablation points of cardiac tissue. Each ablation point includes those features as a time-series sampled sixty (60) times per second, therefore the time-varying nature of each ablation point is also modelled and serves as part of the ablation feature space.

The anatomical structure of each ablation point is also part of the input space of the model. For example, if a point is predicted as being part of a right wide area circumferential ablation (WACA) an ML model for the WACA will classify ablation sites to be one of the following

1. Right Inferior
2. Right Posterior
3. Right Roof
4. Right anterior

For the left WACA each ablation site is associated to one of the following:

1. Left Inferior
2. Left Posterior
3. Left Anterior
4. Left Roof
5. Ridge

An ML model that classifies that way ablation site is provided in U.S. Provisional Patent Application No. 63/059060, titled, "Automatic Segmentation of Anatomical Structures of Wide Area Circumferential Ablation Points," filed July 30, 2020, which is assigned to the assignee of the present patent application.

The disclosed model provides as an output estimates of a lesion surface area (as a radius) 210 and a lesion depth 208, and is operated by a computer processor 206. In operation, the processor applies an algorithm to the built model, comprising inputting data from an ablation procedure, and outputting the lesion estimates 208 and 210.

In this embodiment, a ground truth of the ablation training data used is derived from clinical and preclinical data. Some data is computed from *in vivo* open-chest procedures performed on pigs or/and sheep. In the procedures, lesions are created with a range of powers, to achieve different lesion depths in sheep and pigs in both the atria and the ventricles. The surface area and the depth of tissue necrosis are collected.

In addition, energy may be delivered with a range of powers to achieve different lesion depths in human subjects. The energy delivered to both the atria and the ventricles, and the surface area and the depth of necrosis may be measured using ultrasound/MRI (magnetic resonance imaging).

For both the animal and human subjects, the ablation training data collection includes ECG and intracardiac ECG signals, the ablation catheter type (e.g., focal, lasso, basket, balloon), the 3D location of ablation points, the ablation duration of each point, whether irrigation is used (and, if so, the rate of irrigation), the impedance of the ablation electrode, the power delivered and the temperature profile measured during the ablation. Additional optional data include intracardiac ultrasound, external ultrasound, real time CT, and real time MRI images.

The data referred to above is used as inputs for the random forest regression trees model.

As illustrated in Fig. 2, the model estimates the depth and area of tissue necrosis with two output nodes comprising an average depth of a lesion and a surface area of the lesion, as a radius of the lesion. In an alternative embodiment, a model with three output nodes is generated, where the surface area of the lesion is estimated using major and minor axes of an ellipse.

In all embodiments the estimated surface area of necrosis after each ablation is displayed in real time.

Fig. 3 is a flow chart of training and use for inference, of a machine learning (ML) model to estimate and correct ablation results, according alternative embodiments of the present invention. In particular, the embodiments described by Fig. 3 assist in deciding if, and where, an ablation redo is required in a cardiac chamber.

In one embodiment of the algorithm, the model may be used to identify, after a first "ablation loop," potential locations for an ablation redo, i.e., a repeat ablation.

In a disclosed embodiment of the invention, the model uses a fully connected neural network with an ablation feature space as an input layer. The network has two hidden layers containing rectified linear units (ReLUs) and a single output neuron - a supporting binary first classifier - comprising ablation success or redo. The weights of the network are estimated using a gradient descent optimizer to minimize cross-entropy loss.

In cases where the first classifier predicts a redo, a second classifier marks "low depth" "small surface" lesions as potential places for redoing the ablation.

In another embodiment of the algorithm, which is activated following several ablations but before completion of a first round of PVI, the algorithm identifies potential gaps in a segment of ablation points, e.g., when there is a segment of 50 mm of ablation points near each other, and one of the ablations had a low impedance drop, and the catheter is 20 mm from this segment, the system notifies the physician about these potential gaps.

In yet another embodiment of the algorithm the system notifies in "real time," typically during an ablation, if there is a potential gap within former ablations or if an additional ablation should be performed near the current ablation point.

In a further embodiment of the algorithm the system indicates to the physician where a next point of ablation should be performed. The system also lists ablation parameters to be used, and their values, until completion of the ablation. The algorithm typically determines if completion has been achieved, and notifies the physician accordingly.

The algorithm, according to one presented embodiment, is divided into two parts, algorithm preparation 101 and algorithm use 102.

Algorithm preparation carries out a process that begins at ML modeling step 70, to generate an ML algorithm for estimating ablation results. Such a model can be a supervised ML model, or a reinforcement ML model, variational autoencoder, and general adversarial network, (GAN) among other possible options. The model accepts EP and ablation results as input, among other inputs described below.

Next, using a database comprising ablation training data and ground truth results, a processor trains the algorithm, at an ML algorithm training step 72.

In some embodiments, the ablation training data for the model is split into two categories, data from first ablation sessions that achieves acute success, and data from first ablation sessions that needs a redo procedure (e.g., after finding that the quality of the initial ablation operation does not meet a quality criterion, such as contiguity achieved).

In the disclosed embodiment, the ablation training data may include information taken from actual treatment with a system such as CARTO, e.g.:

    1. Ablation catheter type
    2. 3D location of ablation points
    3. The anatomical location of ablation points
    4. Power used for ablation
    5. Time of point ablation duration
    6. Irrigation
    7. Catheter stability, i.e., the force applied to the catheter during the ablation
    8. Parameters related to the area of the ablation to verify transmural ablation based on the "predicted" tissue width.
    9. 12-lead ECG, or any type of surface ECG, and IcECG
    10. Tissue response, e.g., temperature, ultrasound reflection change, ECG signal reduction, impedance change
    11. External device data alone, e.g., MRI and/or ultrasound data
    12. External device data combined with any of items 1 - 10.

In an alternative disclosed embodiment, the ablation training data may include training data based on images generated from CARTO® (or a similar system). Such images include:

    1. Generated sets of images based on the different CARTO Maps LAT, Voltage, Visitag (e.g., ball-shaped marker on an image, as seen in Fig. 4. The ball size and color indicate the power that was transmitted and the time of transmission), etc. for each one of a procedure phase (Left-Side PVs, and Right-Side PVs), and for different stages of the ablation.
    2. Images taken at several fixed views.
    3. The beginning of the training, including images of the left PV isolation and the right PV isolation.

A second stage of training, including images of a section (a part of a full PVI) isolation.

4. All the map images with the same views, and the images for the training including all CARTO coloring methods: voltage map, LAT maps, bipolar maps

5. Images of all the ablation tag options that are typically generated only for machine learning (including the standard CARTO Visitag/SurPoints/Ablation Index and additional maps that present other parameters such as by coloring the Visitag balls based on catheter stability during the ablation or by coloring the Visitag balls based on any other parameter that may contribute to the machine learning):

- Standard CARTO ablation points
- Points with a color gradient according to any of the following parameters:

  o Supplied energy
  o Catheter force
  o Catheter angle
  o Previous catheter location
  o Ablation power
  o Ablation time
  o Temperature
  o ICEG bipolar drop
  o Catheter stability
  o Respiration during ablation
  o Irrigation

In either of the disclosed embodiments, corrections of a first PVI loop may also be used as part of the training data. These corrections may comprise the following:

a. Parameters of additional ablation points such as ablation catheter type, 3D location of ablation points, power used for ablation, duration of point ablation, irrigation, catheter stability, catheter force, and/or:
b. Images of ablations performed as corrections. The images may include ablation location and supplied energy, if images are taken immediately after a redo.

In addition to the training data described above, ground truth data is also used to build the model, as is illustrated in the figure. The ground truth data is typically based on hospital databases, and may be divided into two sections:

a. Acute success, which is determined by pacing and/or applying an adenosine challenge, so that there is no need for short term follow-up.
b. 12-month follow-up success, which is determined by reviewing redo cases and cases with follow-up information and on clinical research that includes follow-up.

In some embodiments, the ground truth data may comprise effectiveness success and clinical success criteria as defined in the PRECEPT study by Mansour M, et al., titled "Persistent atrial fibrillation ablation with contact force sensing catheter: The prospective multicenter PRECEPT Trial," and published at JACC: Clinical Electrophysiology, Volume 6, Issue 8, August 2020.

In some embodiments, the ground truth data may comprise acquired data that is not presented to a physician because it does not have a known clinical benefit.

While the description above refers to embodiments comprising two separate machine learning models, it will be appreciated that the embodiments may be combined into one model that is implemented to perform the functions of both embodiments.

The algorithm preparation ends with storing the trained model in a non-transitory computer-readable medium, such as a disc on key (memory stick), at a trained model storing step 74. In alternative embodiments, the model is sent in advance, and its optimized parameters (such as weights of an ANN) are sent separately after training.

Algorithm use 102 carries out a process that begins at algorithm uploading step 76, during which a user uploads to a processor either an entire ML model or its optimized parameters (e.g., weights). Next, the processor, such as processor 28, receives patient data similar to the data type used in training, for example, ECGs and EGMs from electrodes 49 and 48, respectively at patient data receiving step 78.

Next, using the trained ML model for inference, the processor inputs data from a selected patient to the model, and implements an algorithm on the model so that the model is able to, for example, output complementary ablation operation (e.g., corrective action) required to make a more contiguous ablation, at ablation recommendations step 80.

After being installed to the processor, the trained model may be used with multiple patients.

The example flow chart shown in Fig. 3 is chosen purely for the sake of conceptual clarity. The present embodiment may also comprise additional steps of the algorithm, such as receiving indications of the degree of physical contact of the electrodes with diagnosed tissue. This and other possible steps are omitted from the disclosure herein purposely in order to provide a more simplified flow chart.

Fig. 4 is screen shot of visualizations of pulmonary veins isolation (PVI) planned using the ML model of Fig. 3, according to an embodiment of the present invention.

Fig. 4 describes an output of an "evaluation engine" (e.g., ML algorithm of Fig. 3), which estimates contiguity and transmurally of PVI. In one embodiment, during the training phase, the evaluation engine has a set of cases tagged by a trained physician with the following information:

1. Ablation anatomical location (Left WACA, Right WACA, Roof line etc.)
2. Ablation type (Effective, ineffective ablation, acute reconnection ablation points or redo ablation point)
3. Width and depth of the ablation

Based on the feature space described above, the ML algorithm provides a prediction per ablation for its effectiveness (e.g., a probability between zero and one), width, and depth of the lesion. The Engine can also recommend on zones of potential acute reconnection sites or long term (redo) reconnection sites.

Discs 402 shown in the figure represent an ablation point, the size (and/or greyscale of the disc) are created automatically by the evaluation engine to depict the contiguity or transmurally of the PVI. The size of the discs can represent ablation effectiveness probability, they can also represent the width and/or depth of the lesion.

In some embodiments, the engine can also give the recommendation to ablate areas 404 to avoid a redo case.

Although the embodiments described herein mainly address cardiac ablation applications, the methods and systems described herein can also be used in other medical applications, such as renal denervation, after re-training with the relevant data input and considering relevant success criteria.

It will be thus appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

**Claims**

1. A system for guiding cardiac ablation, the system comprising:

   an interface configured to receive data that characterizes an initial ablation operation applied to a region of a heart of a patient; and
   a processor, which is configured to automatically specify, based on the received data, a complementary ablation operation to be applied to the region.

2. The system according to claim 1, wherein the processor is configured to specify the complementary ablation by assessing a quality of the initial ablation operation, and specifying the complementary ablation operation in response to finding that the quality of the initial ablation operation does not meet a quality criterion.

3. The system according to claim 1, wherein, in specifying the complementary ablation operation, the processor is configured to specify a location for a repeat ablation.

4. The system according to claim 1, wherein, in specifying the complementary ablation operation, the processor is configured to indicate a gap in a segment of ablation points.

5. The system according to claim 1, wherein, in specifying the complementary ablation operation, the processor is configured to specify, in real time, that an additional ablation is to be performed in proximity to a segment of ablation points.

6. The system according to claim 1, wherein, in specifying the complementary ablation operation, the processor is further configured to specify values of one or more ablation parameters to be used in the complementary ablation.

7. The system according to claim 1, wherein the data that characterizes the initial ablation operation comprises at least one of:

> a body surface electrocardiogram (ECG) signal;
> a change in a body surface ECG signal;
> an intra-cardiac ECG signal;
> a change in an intra-cardiac ECG signal;
> an impedance of an ablation electrode;
> a change in an impedance of an ablation electrode;
> a temperature of ablated tissue;
> a change of temperature of ablated tissue;
> a force on ablated tissue;
> a change of force on ablated tissue;
> an ablation catheter type;
> a 3D location of an ablation point;
> a predicted anatomical location of an ablation point;
> an ablation duration of an ablation point;
> a rate of irrigation; and
> a power delivered during an ablation.

8. The system according to claim 1, wherein the processor is configured to automatically specify the complementary ablation operation by applying a trained machine learning (ML) model.

9. A method for guiding cardiac ablation, the method comprising:

> receiving data that characterizes an initial ablation operation applied to a region of a heart of a patient; and
> automatically specifying, by a processor, based on the received data, a complementary ablation operation to be applied to the region.

10. The method according to claim 9, wherein specifying the complementary ablation comprises assessing a quality of the initial ablation operation, and specifying the complementary ablation operation in response to finding that the quality of the initial ablation operation does not meet a quality criterion.

11. The system according to claim 1 or the method according to claim 9, wherein the data that characterizes the initial ablation operation comprises at least one of:

> a lesion depth;
> a lesion radius;
> a lesion major axis;
> a lesion minor axis;
> a lesion 3D location;
> a lesion anatomical location; and
> a lesion surface area.

12. The method according to claim 9, wherein specifying the complementary ablation operation comprises specifying a location for a repeat ablation.

13. The method according to claim 9, wherein specifying the complementary ablation operation comprises indicating a gap in a segment of ablation points.

14. The method according to claim 9, wherein specifying the complementary ablation operation comprises specifying, in real time, an additional ablation that is to be performed in proximity to a segment of ablation points.

15. The method according to claim 9, wherein specifying the complementary ablation operation comprises specifying values of one or more ablation parameters to be used in the complementary ablation operation.

16. The method according to claim 9, wherein the data that characterizes an initial ablation operation comprises at least one of:

> a body surface electrocardiogram (ECG) signal;
> a change in a body surface ECG signal;
> an intra-cardiac ECG signal;
> a change in an intra-cardiac ECG signal;
> an impedance of an ablation electrode;
> a change in an impedance of an ablation electrode;
> a temperature of ablated tissue;
> a change of temperature of ablated tissue;
> a force on ablated tissue;
> a change of force on ablated tissue;
> an ablation catheter type;
> a 3D location of an ablation point;
> a predicted anatomical location of an ablation point;
> an ablation duration of each point;
> a rate of irrigation; and
> a power delivered during an ablation.

17. The system according to claim 7 or the method according to claim 16, wherein the data that characterizes the initial ablation operation comprises one or both of:

> a change in ultrasound reflection of ablated tissue; and
> a change in a magnetic resonance image (MRI) of ablated tissue.

18. The method according to claim 9, wherein automatically specifying the complementary ablation operation comprises applying a trained machine learning (ML) model.

19. The system according to claim 8 or the method according to claim 16, wherein the ML model comprises at least one of autoencoder, variational autoencoder, general adversarial network (GAN), random forest (RF), supervised ML, and reinforcement ML.

FIG. 1

EP 3 795 078 A1

FIG. 2

```
         ┌─────────────────────────────────────────┐
         │  Generate ML algorithm that accepts       │⟋∿70
         │ electrophysiological and ablation data as input │
         └─────────────────────────────────────────┘
                            │
                            ▼
Preparation ┌─────────────────────────────────────────┐
   101      │ Train the ML algorithm using ablation training │⟋∿72
            │       data and ground truth data           │
            └─────────────────────────────────────────┘
                            │
                            ▼
            ┌─────────────────────────────────────────┐
            │   Store the trained ML algorithm in non-   │⟋∿74
            │     transitory computer-readable medium    │
            └─────────────────────────────────────────┘
                            │
                            ▼
            ┌─────────────────────────────────────────┐
            │       Upload ML algorithm to processor     │⟋∿76
            └─────────────────────────────────────────┘
                            │
                            ▼
   Use      ┌─────────────────────────────────────────┐
   102      │  Receive patient data comprising ablation  │⟋∿78
            │                  results                   │
            └─────────────────────────────────────────┘
                            │
                            ▼
            ┌─────────────────────────────────────────┐
            │   Using the trained ML algorithm estimate  │⟋∿80
            │   corrections required to ablation results │
            └─────────────────────────────────────────┘
```

*FIG. 3*

*FIG. 4*

Europäisches
Patentamt

European
Patent Office

Office européen
des brevets

# EUROPEAN SEARCH REPORT

Application Number

EP 20 19 7183

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/092071 A1 (NAVIX INTERNATIONAL LTD; BEN HAIM SHLOMO [GB] ET AL.) 24 May 2018 (2018-05-24) * page 37, line 6 - page 43, line 2; figures 1a, 4a * * page 90, line 9 - page 94, line 6; claims 1-8,12,20,21; figure 12 * ----- | 1-19 | INV. A61B5/318 ADD. A61B5/0538 A61B5/00 |
| X | US 2018/125575 A1 (SCHWARTZ YITZHACK [IL] ET AL) 10 May 2018 (2018-05-10) * the whole document * ----- | 1,9 | |
| A | US 2019/254564 A1 (SCHWARTZ YITZHACK [IL] ET AL) 22 August 2019 (2019-08-22) * paragraph [0082]; claims 1,11-14,19; figures 4,5 * ----- | 1,9 | |

TECHNICAL FIELDS
SEARCHED (IPC)

A61B

The present search report has been drawn up for all claims

| Place of search | Date of completion of the search | Examiner |
|---|---|---|
| Berlin | 25 January 2021 | Kronberger, Raphael |

EPO FORM 1503 03.82 (P04C01)

**ANNEX TO THE EUROPEAN SEARCH REPORT
ON EUROPEAN PATENT APPLICATION NO.**

EP 20 19 7183

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2021

| Patent document cited in search report | | | Publication date | Patent family member(s) | | | Publication date |
|---|---|---|---|---|---|---|---|
| WO 2018092071 | A1 | | 24-05-2018 | CN | 110198680 | A | 03-09-2019 |
| | | | | EP | 3541313 | A1 | 25-09-2019 |
| | | | | US | 2020022649 | A1 | 23-01-2020 |
| | | | | US | 2020060757 | A1 | 27-02-2020 |
| | | | | WO | 2018092071 | A1 | 24-05-2018 |
| US 2018125575 | A1 | | 10-05-2018 | CN | 107635503 | A | 26-01-2018 |
| | | | | EP | 3294179 | A1 | 21-03-2018 |
| | | | | JP | 2018522612 | A | 16-08-2018 |
| | | | | RU | 2017140235 | A | 13-06-2019 |
| | | | | US | 2018125575 | A1 | 10-05-2018 |
| | | | | WO | 2016181318 | A1 | 17-11-2016 |
| US 2019254564 | A1 | | 22-08-2019 | EP | 3294127 | A1 | 21-03-2018 |
| | | | | US | 2018153437 | A1 | 07-06-2018 |
| | | | | US | 2019254564 | A1 | 22-08-2019 |
| | | | | WO | 2016181316 | A1 | 17-11-2016 |

EPO FORM P0459

For more details about this annex : see Official Journal of the European Patent Office, No. 12/82

## EP 3 795 078 A1

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62903850
- US 9463072 B
- US 9277970 B
- US 8456182 B
- US 63059060

**Non-patent literature cited in the description**

- **MANSOUR M et al.** Persistent atrial fibrillation ablation with contact force sensing catheter: The prospective multicenter PRECEPT Trial. *JACC: Clinical Electrophysiology,* August 2020, vol. 6 (8